(19) Europäisches Patentamt
European Patent Office
Office européen des brevets

(11) Veröffentlichungsnummer: **0 280 290 B1**

(12) **EUROPÄISCHE PATENTSCHRIFT**

(45) Veröffentlichungstag der Patentschrift: **08.01.92**

(21) Anmeldenummer: **88102785.8**

(22) Anmeldetag: **25.02.88**

(51) Int. Cl.5: **A61K 31/55**, A61K 31/44, A61K 31/40, A61K 31/495, A61K 31/415, A61K 31/535, //(A61K31/55,31:44), (A61K31/55,31:40),(A61K31/55, 31:495),(A61K31/55,31:415), (A61K31/55,31:535), (A61K31/44,31:335), (A61K31/44,31:135), (A61K31/40,31:335),A61K31:40, A61K31:135,A61K31:495, A61K31:335

# (54) Mittel mit antidepressiver Wirkung.

(30) Priorität: **27.02.87 DE 3706399**

(43) Veröffentlichungstag der Anmeldung: **31.08.88 Patentblatt 88/35**

(45) Bekanntmachung des Hinweises auf die Patenterteilung: **08.01.92 Patentblatt 92/02**

(84) Benannte Vertragsstaaten: **AT BE CH DE ES FR GB GR IT LI LU NL SE**

(56) Entgegenhaltungen: **EP-A- 0 136 658**

(73) Patentinhaber: **BOEHRINGER INGELHEIM KG**
**Postfach 200**
**W-6507 Ingelheim am Rhein(DE)**
(84) Benannte Vertragsstaaten: **BE CH DE ES FR GR IT LI LU NL SE AT**

Patentinhaber: **BOEHRINGER INGELHEIM INTERNATIONAL GmbH**
**Postfach 20**
**W-6507 Ingelheim am Rhein(DE)**
(84) Benannte Vertragsstaaten: **GB**

**EP 0 280 290 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

**DIALOG 06205426, Medline 87179426; M. YURDAKOK et al.: "Treatment of enuresis: a study with imipramine, amitriptyline, chlordiazepoxide-clidinium and piracetam", & TURK. J. PEDIATR. (TURKEY), Jul-Sep 1986, 28(3), p171-5**

(72) Erfinder: **Lehr, Erich, Dr.**
**In der Toffel 5**
**W-6531 Waldalgesheim(DE)**
Erfinder: **Bechtel, Wolf-Dietrich, Dr.**
**Mühlstrasse 3**
**W-6531 Appenheim(DE)**
Erfinder: **Schuster, Astrid, Dipl.-Chem.**
**In der Rheingewann 23**
**W-6507 Ingelheim/Rhein(DE)**

## Beschreibung

Die Erfindung betrifft ein Mittel mit antidepressiver Wirkung enthaltend eine Verbindung A und eine Verbindung B.

Aus der Literatur ist eine Reihe von tricyclischen Antidepressiva bekannt, die als zentrales Strukturelement ein 10,11-Dihydro-dibenzo[b,f]-azepingerüst, ein Dibenzo[a,d][1,4]cycloheptadiengerüst oder ein [10,11]-Dihydro-dibenzo[b,f]oxepingerüst enthalten.

Einerseits besitzen diese Verbindungen hervorragende antidepressive Eigenschaften und haben sich in der Therapie bewährt, andererseits ist die therapeutische Anwendungsbreite aufgrund von unerwünschten Nebeneffekten, wie z.B. Cardiotoxität, proconvulsiver Wirkung, doch erheblich eingeschränkt.

Überraschenderweise wurde nun gefunden, daß durch die Kombination eines Antidepressivums vom Strukturtyp eines 10 11-Dihydro-dibenzo[b,f]azepins (A), eines Dibenzo[a,d][1,4]cycloheptadiens und/oder eines [10,11]-Dihydro-dibenzo[b,f]oxepingerüst sowie gegebenenfalls deren pharmakologisch verträgliche Säureadditionssalze und einer Verbindung mit der Struktur eines N-Benzyl-Pyrrolidin-2-ons (B) unerwünschte Nebenwirkungen erheblich vermindert werden, die antidepressiven Wirkungen hingegen sogar noch deutlich gesteigert wurden.

Verbindungen des Strukturtyps B sind beispielsweise 1-Benzyl-aminomethyl-pyrrolidin-2-one bzw. 1-Pyridyl-aminomethyl-pyrrolidin-2-one der allgemeinen Formel I

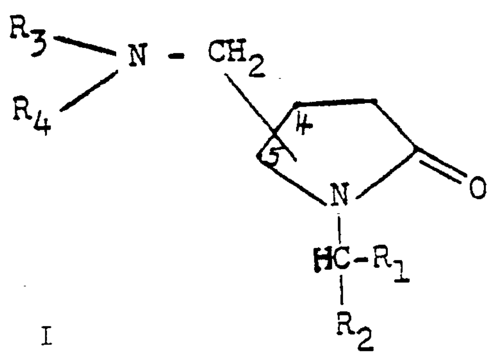

I

worin

$R_1$ Wasserstoff oder einen Alkylrest,

$R_2$ einen phenylrest, der ein- oder zweifach durch Alkoxy, Fluor, Chlor, Brom, Trifluormethyl, Alkyl, Hydroxy oder Nitro substituiert sein kann oder einen Pyridylrest und

$R_3$ Wasserstoff oder einen Alkylrest;

$R_4$ Wasserstoff oder einen Alkylrest

bedeuten oder beide Reste $R_3$ und $R_4$ zusammen mit dem Stickstoffatom einen gesättigten 5- oder 6-Ring, der als weiteres Heteroatom ein O- oder N-Atom enthalten und gegebenenfalls durch Alkyl, bevorzugt Methyl substituiert sein kann oder einen Imidazolring bilden und wobei die Aminoalkylgruppe in 4- oder 5-Stellung steht und deren pharmakologisch verträgliche Säureadditonssalze,

In der allgemeinen Formel I steht die Bezeichnung "Alkyl" für eine geradkettige oder verzweigte Alkylgruppe mit 1 -4 Kohlenstoffatomen, wie z.B. Methyl, Ethyl, Propyl, Isopropyl, n-Butyl oder tert.-Butyl und die Bezeichnung "Alkoxy" für einen Rest mit 1 - 2 Kohlenstoffatomen; der als Bedeutung für $R_2$ genannte Pyridylring kann in 2-, 3- oder 4-Stellung mit der Methylenbrücke verknüpft sein.

Verbindungen der allgemeinen Formel I und Verfahren zu ihrer Herstellung sind aus der europäischen Patentanmeldung 136 658 bekannt, wobei die Wirksamkeit der Verbindungen bei Zustandsformen eingeschränkter cerebraler Leistungsfähigkeit offenbart wird.

Bevorzugt sind Verbindungen der allgemeinen Formel I, bei denen $R_1$ Wasserstoff, $R_2$ einen gegebenenfalls durch Fluor, Chlor, Methyl oder Methoxy in o- oder bevorzugt p-Stellung substituierten Phenylrest und $R_3$ und $R_4$ Wasserstoff, Methyl oder Ethyl oder $R_3$ und $R_4$ zusammen Morpholin, N-Methylpiperazin oder Pyrrolidin bedeuten.

Geeignete Verbindungen sind weiterhin:

1-(3,4-Dimethoxybenzyl)-4-aminomethyl-pyrrolidin-2-on

1-(4-Methylbenzyl)-4-aminomethyl-pyrrolidin-2-on

1-(3-Trifluormethylbenzyl)-4-aminomethyl-pyrrolidin-2-on

1-(α-Methylbenzyl)-4-aminomethyl-pyrrolidin-2-on
1-Benzyl-4-piperidinomethyl-pyrrolidin-2-on
1-Benzyl-4-(N-methylpiperazinomethyl)-pyrrolidin-2-on
1-Benzyl-4-(imidazol-1-yl-methyl)-pyrrolidin-2-on
1-Benzyl-4-methylaminomethyl-pyrrolidin-2-on
1-(p-Fluorbenzyl)-4-dimethylaminomethyl-pyrrolidin-2-on
1-(4-Nitrobenzyl)-4-aminomethyl-pyrrolidin-2-on
1-(4-Hydroxybenzyl)-4-aminomethyl-pyrrolidin-2-on
1-(o-Chlorbenzyl)-4-aminomethyl-pyrrolidin-2-on
1-(o-Chlorbenzyl)-4-diethylaminomethyl-pyrrolidin-2-on
1-Benzyl-4-isopropylaminomethyl-pyrrolidin-2-on
1-(p-Methylbenzyl)-4-diethylaminomethyl-pyrrolidin-2-on
1-Benzyl-5-dimethylaminomethyl-pyrrolidin-2-on
1-Benzyl-5-diethylaminomethyl-pyrrolidin-2-on
1-Benzyl-5-morpholinomethyl-pyrrolidin-2-on
1-Benzyl-5-(4-methylpiperazino)-methyl-pyrrolidin-2-on
1-Benzyl-5-pyrrolidinomethyl-pyrrolidin-2-on
1-(4-Methylbenzyl)-5-dimethylaminomethyl-pyrrolidin-2-on
1-(4-Methylbenzyl-5-diethylaminomethyl-pyrrolidin-2-on
1-(p-Chlorbenzyl)-5-dimethylaminomethyl-pyrrolidin-2-on
1-(p-Chlorbenzyl)-5-diethylaminomethyl-pyrrolidin-2-on
1-(3,4-Dichlorbenzyl)-5-dimethylaminomethyl-pyrrolidin-2-on
1-(3,4-Dichlorbenzyl)-5-diethylaminomethyl-pyrrolidin-2-on
1-(p-Methoxybenzyl-5-dimethylaminomethyl-pyrrolidin-2-on
1-(p-Methoxybenzyl)-5-diethylaminomethyl-pyrrolidin-2-on
1-Benzyl-5-aminomethyl-pyrrolidin-2-on

Bevorzugte Verbindungen sind:
1-(4-Methoxybenzyl)-4-aminomethyl-pyrrolidin-2-on
1-Benzyl-4-N,N-diethylaminomethyl-pyrrolidin-2-on
1-(4-Fluorbenzyl)-4-aminomethyl-pyrrolidin-2-on
1-(4-Chlorbenzyl)-4-aminomethyl-pyrrolidin-2-on
1-(4-Pyridylmethyl)-4-aminomethyl-pyrrolidin-2-on
1-(4-Fluorbenzyl)-4-(morpholinomethyl)-pyrrolidin-2-on
1-Benzyl-4-(N-methylpiperazinylmethyl)-pyrrolidin-2-on
1-Benzyl-4-methylaminomethyl-pyrrolidin-2-on.

Ganz besonders bevorzugt ist
1-Benzyl-4-aminomethyl-pyrrolidin-2-on

Bevorzugte Verbindungen des Strukturtyps A sind Desipramin. Clomipramin, Amitriptylin, Nortriptyline, Maprotilin und Doxepin.

Besonders bevorzugt ist Imipramin, ein 10,11-Dihydro-N,N-dimethyl-5H-dibenz[b,f]azepin-5-propanamin

Das erfindungsgemäße Mittel kann als Kombination eine oder mehrere Verbindungen A und eine odere mehrere Verbindungen B enthalten. Bevorzugt sind Kombinationen, die eine Verbindung A und eine Verbindung B enthalten.

Als weitere Bestandteile kann das erfindungsgemäße Mittel die üblichen galenischen Hilfsstoffe und Trägerstoffe enthalten.

Bevorzugt ist die Kombination von Imipramin und 1-Benzyl-4-aminomethl-pyrrolidin-2-on.

Durch die erfindungsgemäße Kombination herkömmlicher Antidepressiva (A) mit 1-Benzyl- bzw. 1-Pyridyl-amino-methylpyrrolidin-2-onen, insbesondere von 1-Benzyl-4-aminomethyl-pyrrolidin-2-on, wird die toxische Wirkung der herkömmlichen Antidepressiva erheblich reduziert.

Ein empfindlicher Test für den praeklinischen Wirkungsnachweis antidepressiver Eigenschaften ist der Küken-Ruftest. Dabei gilt die im Testverlauf abnehmende Rufhäufigkeit isolierter Eintagsküken als tierexperimentelles Verhaltensmodell zu Resignationserscheinungen bei der Depression. Das Verfahren konnte mit dem Test zahlreicher neurotrop wirksamer Substanzen validiert werden; es zeichnet sich durch gut reproduzierbare Selektivität für bereits klinisch bewährte Antidepressiva aus, welche dosisabhängig die abgesunkene Rufrate zu reaktivieren vermögen (Distress call activation in isolated chicks; A new behavioural model for antidepressants, E. Lehr, Psychopharmacol. 89, 21 (1986); Aktivierung des Kontaktrufens als tierexperimentelles Verhaltensmodell zur Depressionsforschung. E. Lehr, Fortschr. Neurol. Psychiat. 54, 26 (1986).

In der Tabelle I sind die pharmakologischen Daten für Imipramin (Verbindung A), 1-Benzyl-4-aminomethyl-pyrrolidin-2-on (Fumarat) (Verbindung B) und die Kombination von A + B aufgeführt.

Tab.: I Pharmakologische Testergebnisse

| Test | Verb. A | Verb. B | Kombination A + B |
|---|---|---|---|
| Isolierte Küken | 10 mg/kg i.p. | 5 mg/kg i.p. | 10 mg/kg A/ 5 mg/kg B |
| Steigerung der Referate i.d. 2. Stunde | 131 % | 284 % | 412 % |
| Placebo = 100 % | | | |
| Maus | | | |
| Hemmung der durch Tetrabenazin induz. | 3 mg/kg p.o. | 3,5 mg/kg p.o. | 3 mg/kg A + 3,5 mg/kg B |
| Ptosis | 12 % | 48 % | 88 % |
| Kontrolle ohne Tetrabenazin = 100 % | | | |

Die erfindungsgemäße Kombination wurde hinsichtlich einer möglichen Potenzierung der toxischen Wirkung nach intravenöser Applikation an Mäusen geprüft.

Versuchsziel:

Ermittlung der Verhaltenänderungen, Vergiftungssymptome und eventuell der Zielorgane der toxischen Wirkung nach unmittelbar aufeinanderfolgender Gabe subletaler Dosen von Imipramin-HCl und 1-Benzyl-4-aminomethyl-pyrrolidin-2-on FU, sowie Erfassung einer Potenzierung der toxischen Wirkung.

Versuchtiere:

Albinomäuse des Stammes Chbb: NMRI

| | | |
|---|---|---|
| Alter bei Versuchsbeginn: Tage | m | 40 - 46 |
| | f | 40 - 46 |

Gewicht (Mittelwerte bei Versuchsbeginn):

| | |
|---|---|
| m | ca. 27 g |
| f | ca. 24 g |

Es wurde eine 2%ige Lösung 1-Benzyl-4-aminomethyl-pyrrolidin-2-on Fumarat sowie eine 0,25%ige Imipramin-Lösung (pH = 6,2) verwendet. Die Versuchsergebnisse sind in Tab.: II wiedergegeben.

Tab. II

| Gruppe (m und f) | Prüfsubstanz | Dosis mg/kg | Letalität tote/behandelte Tiere |
|---|---|---|---|
| 1 | 1-Benzyl-4-aminomethyl-pyrrolidin-2-on-FU | 100 | 0/10 |
| 2 | Imipramin-HCl | 12,5 | 0/10 |
| 3* | Imipramin-HCl + | 12,5 | |
| | 1-Benzyl-4-aminomethyl-pyrrolidin-2-on-FU | 100 | 0/10 |

* Die Tiere der Gruppe 3 wurden zuerst mit Imipramin und unmittelbar danach (ca. 10-15 Sekunden) mit 1-Benzyl-4-aminomethyl-pyrrolidin-2-onFU behandelt.

DOSISBEGRÜNDUNG

Die $LD_{50}$ i.v. Maus für Imipramin beträgt nach Literaturangaben 21 bzw. 36 mg/kg (Archives International de Pharmacodynamie et de Therapie. 144, 481. 1963 bzw. 245, 283, 1980). In einem Vorversuch starb bei 16 mg/kg eines von 4 behandelten Tieren, bei 12.5 mg/kg wurde kein Todesfall registriert. Bei 12,5 mg/kg ist demnach zwar eine Wirkung jedoch kein Todesfall zu erwarten.

Durch die gleichzeitige Gabe von Imipramin und 1-Benzyl-4-aminomethyl-pyrrolidin-2-onFU in der maximalen, nichtletalen Dosierung, die im Bereich der Hälfte der $LD_{50}$ der Einzelsubstanzen liegt oder diesen sogar überschreitet, konnte jedoch gezeigt werden, daß die erfindungsgemäße Kombination nicht zu einer Potenzierung der Toxizität führt.

Weiterhin wurde die antagonistische Wirkung im toxischen Dosisbereich von Imipramin nach intravenöser Applikation der Kombination an Mäusen geprüft.

Versuchsziel:

Ermittlung der Verhaltensänderungen, Vergiftungssymptome und eventuell der Zielorgane nach unmittelbar aufeinanderfolgender Gabe subletaler Dosen von 1-Benzyl-4-aminomethyl-pyrrolidin-2-onFU und letaler Dosen von Imipramin-HCl, sowie die Erfassung einer antagonistischen Wirkung. Die Versuchsergebnisse sind Tab. III wiedergegeben.

Prüflösungen:

Die Prüfsubstanzen wurden in 0,9%iger NaCl-Lösung gelöst.

Es wurden 1,5%ige 1-Benzyl-4-aminomethyl-pyrrolidin-2-onFU-Lösungen (pH = 6,4) sowie 0,5 %ige Imipramin-Lösungen (pH = 5,9) verwendet.

| Gruppe (m und f) | 1-Benzyl-4-aminomethyl-pyrrolidin-2-onFU mg/kg | Imipramin mg/kg | Letalität tote/ behandelte Tiere |
|---|---|---|---|
| 1 | - | 25 | 4/10 |
| 2** | 75 | 25 | 2/10 |

** Die Tiere der Gruppe 2 wurden zuerst mit 1-Benzyl-4-aminomethyl-pyrrolidin-2-onFU und unmittelbar danach (ca. 10 - 15 Sek.) mit Imipramin behandelt.

Die $LD_{50}$ i.v. Maus für Imipramin beträgt laut Literaturangaben 21 bzw. 36 mg/kg (siehe oben). Die Dosis von 12,5 mg/kg erwies sich als maximale nicht letale Dosis. Nach intravenöser Gabe von 25 mg/kg wurde für Imipramin mit einer letalen Wirkung gerechnet, wie es durch die Versuchsergebnisse auch bestätigt wurde.

Die unmittelbar aufeinanderfolgenden intravenösen Gaben einer subletalen Dosis von 1-Benzyl-4-aminomethyl-pyrrolidin-2-onFU und einer sicher letalen Dosis von Imipramin führte überraschenderweise zu einer verringerten Sterblichkeitsrate der Tiere.

Die vorliegenden Versuchsergebnisse zeigen eine Verminderung der toxischen Wirkung von Imipramin nach Vorbehandlung der Tiere mit 1-Benzyl-4-aminomethyl-pyrrolidin-2-on.

Aufgrund der in den Tabellen wiedergegebenen Daten läßt sich ein eindeutiger Synergismus der erfindungsgemäßen Kombination nachweisen, der eine deutliche Überlegenheit der Kombination in der antidepressiven Wirkung mit gleichzeitiger Verringerung der unerwünschten Nebenwirkungen in Bezug zu den Einzelverbindungen aufzeigt.

Verfahren zur Herstellung von Verbindungen des Strukturtyps A sind Stand der Technik.

Verfahren zur Herstellung von Verbindungen der allgemeinen Formel I sowie ihrer pharmakologisch verträglichen Säureadditonssalze sind ebenfalls bekannt und in der europäischen Patentanmeldung 136 658 beschrieben, auf die hiermit inhaltlich Bezug genommen wird.

Die erfindungsgemäße Kombination [A und B] kann für sich allein oder gegebenenfalls in weiterer Kombination mit anderen weiteren pharmakologisch aktiven Wirkstoffen zur Anwendung gelangen. Geeignete Anwendungsformen sind beispielsweise Tabletten, Kapseln, Zäpfchen, Lösungen, Säfte, Emulsionen oder dispersible Pulver. Entsprechende Tabletten können beispielsweise durch Mischen des oder der Wirkstoffe mit bekannten Hilfsstoffen, beispelsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk und/oder Mitteln zur Erzielung des Depoteffektes, wie Carboxypolymethyylen, Carboxymethylcellulose. Celluloseacetatphthalat, oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen, wobei die oben bei den Tabletten erwähnten Hilfsstoffe verwendet werden können.

Säfte der erfindungsgemäßen Wirkstoffkombinationen können zusätzlich noch ein Süßungsmittel wie Saccharin, Cyclamat, Glycerin oder Zucker sowie ein geschmacksverbesserndes Mittel, z.B. Aromastoffe, wie Vanillin oder Orangenextrakt, enthalten. Sie können außerdem Suspendierhilfsstoffe oder Dickungsmittel, wie Natriumcarboxymethylcellulose, Netzmittel, beispielsweise Kondensationsprodukte von Fettalkoholen mit Äthylenoxid, oder Schutzstoffe, wie p-Hydroxybenzoate, enthalten.

Injektionslösungen werden in üblicher Weise, z.B. unter Zusatz von Konservierungsmitteln, wie p-Hydroxybenzoaten, oder Stabilisatoren, wie Alkalisalzen oder Äthylendiamintetraessigsäure hergestellt und in Injektionsflaschen oder Ampullen abgefüllt.

Die eine erfindungsgemäße Wirkstoffkombination enthaltenden Kapseln können beispielsweise hergestellt werden, indem man die Wirkstoffe mit inerten Trägern, wie Milchzucker oder Sorbit, mischt und in Gelatinekapseln einkapselt. Geeignete Zäpfchen lassen sich beispielsweise durch Vermischen mit dafür vorgesehenen Trägermitteln, wie Neutralfetten oder Polyäthylenglykol beziehungsweise dessen Derivaten, herstellen.

Die Dosierung einer therapeutisch wirksamen Menge der Kombination liegt im allgemeinen zwischen 5 und 500 mg, bevorzugt zwischen 20 und 200 mg je Einzeldosis.

Die nachfolgenden Beispiele illustrieren die vorliegende Erfindung,:

Beispiel 1

1-Benzyl-4-aminomethyl-pyrrolidin-2-on

54 g (0,16 Mol) 4-Phthalimidomethyl-1-benzyl-pyrrolidin-2-on werden in 1,3 1 Äthylalkohol nach Zusatz von 32 g Hydrazinhydrat 4 Stunden bei Raumtemperatur gerührt. Der Niederschlag (Phthalsäurehydrazid) wird abgesaugt und das Filtrat eingedampft. Man setzt 500 ml Methylenchlorid zu dem Rückstand und schüttelt dreimal mit 100 ml Wasser aus. Die organische Phase wird getrocknet und eingedampft. Den verbleibenden Rückstand löst man in 500 ml Methanol und fügt bei Siedehitze unter Rühren 20 g (0.17 Mol) feste Fumarsäure portionsweise hinzu. Beim Abkühlen scheiden sich farblose Kristalle ab. die man absaugt und mit Methanol und Äther nachwäscht.
Ausbeute: 20 - 25 g (48 - 60 % d.Th.)
vom Fp. 209 - 211 ° C.
Die Verbindung enthält 1/2 Mol Fumarsäure.

Das Ausgangsmaterial wird wie folgt erhalten:

a) 94 g (0,46 Mol) 1-Benzyl-4-hydroxymethyl-pyrrolidin-2-on werden mit 700 ml Methylenchlorid und 40 ml (0.54 Mol) Thionylchloid 25 Stunden unter Rückflußkochen gerührt und das Reaktionsgemisch anschließend unter Kühlung mit verdünntem Ammoniak neutralisiert. Nach Abtrennen, Trocknen und Eindampfen hinterbleiben 85 - 90 g eines dunklen Öls, welches direkt zur weiteren Umsetzung verwendet wird.

b) 43.5 g (0,195 Mol) rohes 1-Benzyl-4-chlormethyl-pyrrolidin-2-on, 36 g (0,195 Mol) Phthalimidkalium unu 700 ml Dimethylformamid werden 2 Stunden unter Rückfluß gekocht. Das Reaktionsgemisch wird anschließend im Vakuum eingedampft und der Rückstand in Methylenchlorid aufgenommen. Man schüttelt mehrmals mit Wasser aus, trocknet die organische Phase und erhält nach Chromatographie über $SiO_2$ 45 g (70 % d.Th.) der Phthalimidoverbindung vom Fp. 108 - 109 ° C.

Beispiel 2

1-Benzyl-4-aminomethyl-pyrrolidin-2-on

a) 58 g (0,29 Mol) 1-Benzyl-4-nitrilo-pyrrolidin-2-on werden in Methanol gelöst und unter Zusatz von flüssigem Ammoniak über Raney-Nickel katalytisch hydriert. Nach dem Einengen der Reaktionslösung wird in Methanol gelöst, von Katalysatorresten abfiltriert und nach dem Erwärmen auf ca. 50 ° C mit 17 g Furmarsäure versetzt. Unter Rühren geht die Fumarsäure kurzzeitig in Lösung, danach beginnt die Kristallisation des 1-Benzyl-4-aminomethyl-pyrrolidin-2-on-fumarats.
Ausbeute: 68 g (= 91 % d.Th.); Fp. 192 - 194 ° C.

b) Die Nitriloverbindung wird in 96 % Ausbeute aus dem entsprechenden Amid vom Fp. 162 - 166 ° C durch Dehydratisierung mittels $POCl_3$ in Dimethylformamid bei ca. 60 ° C als Öl erhalten.

Beispiel 3

Racematspaltung von 1-Benzyl-4-aminomethyl-pyrrolidin-2-on

a) 24,0 g (0,117 Mol) 1-Benzyl-4-aminomethyl-pyrrolidin-2-on werden in 200 ml heißem Methanol gelöst, 17,6 g (0,117 Mol) L(+)-Weinsäure werden ebenfalls in 200 ml heißem Methanol gelöst. Die beiden Lösungen werden zusammengegeben und unter Rühren auf Raumtemperatur abkühlen lassen, wobei das Salz auskristallisiert. Die Kristalle werden kalt abgesaugt, mit kaltem Methanol nachgewaschen und getrocknet. Ausbeute: 18,0 g 4-Aminomethyl-1-benzyl-pyrrolidin-2-on-tartrat. Fp. 204 - 206 ° C (aus Methanol),
$\alpha_D$ = -6,3 ° (c = 1,0; Wasser)-

b) Zur Umwandlung des Tartrats in die Base wird das Tartrat kalt in 20 ml Wasser und 10 ml konzentrierter Natronlauge gelöst, dreimal mit Methylenchlorid extrahiert, die vereinigten Methylenchloridphasen über $MgSO_4$ getrocknet und das Lösungsmittel i.V. abgezogen.
Man erhält das (-)-4-Aminomethyl-1-benzyl-pyrrolidin-2-on, $\alpha_D$ = -8,4 ° (c = 1.0; Wasser).

c) Die bei der unter a) angegebenen Aufarbeitung anfallenden Mutterlaugen werden i.V. eingeengt. Man erhält 38,0 g des Tartrats, das kalt in 140 ml Wasser und 50 ml konzentrierter Natronlauge aufgenommen und dreimal mit Methylenchlorid ausgeschüttelt wird. Die vereinigten Methylenchloridphasen werden über $MgSO_4$ getrocknet und das Lösungsmittel i.V. abgezogen. Man erhält: 19,3 g Base, die, wie

unter a) beschrieben, mit D-(-)-Weinsäure in das entsprechende Tartrat überführt wird. Ausbeute: 19,0 g vom Fp. 204 - 205 °C.

d) Die Umwandlung des Tartrats in die Base erfolgt wie unter b) beschrieben.

Man erhält 5,7 g (+)-4-Aminomethyl-1-benzyl-pyrrolidin-2-on mit einem Drehwert $\alpha_D = +8,4^\circ$ (c = 1,0; Wasser).

Pharmazeutische Formulierungsbeispiele

| A) Tabletten | pro Tablette |
|---|---|
| Wirkstoff A + B | 100 mg |
| Milchzucker (pulverisiert) | 140 mg |
| Maisstärke | 240 mg |
| Polyvinylpyrrolidon | 15 mg |
| Magnesiumstearat | 5 mg |
| | 500 mg |

Der feingemahlene Wirkstoff, Milchzucker und ein Teil der Maisstärke werden miteinander vermischt. Die Mischung wird gesiebt, worauf man sie mit einer Lösung von Polyvinylpyrrolidon in Wasser befeuchtet, knetet, feuchtgranuliert und trocknet. Das Granulat, der Rest der Maisstärke und das Magnesiumstearat werden gesiebt und miteinander vermischt. Das Gemisch wird zu Tabletten geeigneter Form und Größe verpreßt.

| B) Tabletten | pro Tablette |
|---|---|
| Wirkstoff A + B | 80 mg |
| Maisstärke | 190 mg |
| Milchzucker | 55 mg |
| Mikrokristalline Cellulose | 35 mg |
| Polyvinylpyrrolidon | 15 mg |
| Natrium-Carboxymethylstärke | 23 mg |
| Magnesiumstearat | 2 mg |
| | 400 mg |

Der feingemahlene Wirkstoff, ein Teil der Maisstärke, Milchzucker, mikrokristalline Cellulose und Polyvinylpyrrolidon werden miteinander vermischt, die Mischung gesiebt und mit dem Rest der Maisstärke und Wasser zu einem Granulat verarbeitet, welches getrocknet und gesiebt wird. Dazu gibt man die Natrium-carboxymethylstärke und das Magnesiumstearat, vermischt und verpreßt das Gemisch zu Tabletten geeigneter Größe.

| C) Ampullen | |
|---|---|
| 1-Benzyl-4-aminomethyl-pyrrolidin-2-on-Fumarat | 50,0 mg |
| Imipramin | 25,0 mg |
| Natriumchlorid | 10,0 mg |
| bidestilliertes Wasser q.s. ad | 1,0 ml |

Herstellung:

Der Wirkstoff und das Natriumchlorid werden in bidestilliertem Wasser gelöst und die Lösung in Ampullen steril abgefülllt.

| D) Tropfen | |
|---|---|
| 1-Benzyl-4-aminomethyl-pyrrolidin-2-on-Fumarat | 5,0 mg |
| Imipramin | 2,5 mg |
| p-Hydroxybenzoesäuremethylester | 0,1 g |
| p-Hydroxybenzoesäurepropylester | 0,1 g |
| entmineralisiertes Wasser q.s. ad | 100,0 ml |

Herstellung

Der Wirkstoff und die Konservierungsmittel werden in demineralisiertem Wasser gelöst und die Lösung filtriert und in Flaschen zu je 100 ml abgefüllt.

**Patentansprüche**

1. Mittel enthaltend ein Antidepressivum [A] des Strukturtyps eines 10,11-Dihydro-dibenzo[b,f]azepins, eines Dibenzo[a,d][1,4]cycloheptadiens und/oder eines [10,11]-Dihydro-dibenzo-[b,f]oxepins sowie gegebenenfalls deren verträgliche Säureadditionssalze und eine Verbindung der allgemeinen Formel I [B]

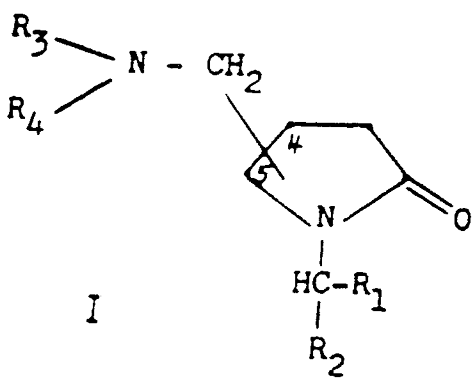

I

worin

$R_1$ Wasserstoff oder einen Alkylrest,
$R_2$ einen Phenylrest, der durch Alkoxy, Fluor, Chlor, Brom, Trifluormethyl, Alkyl, Hydroxy oder Nitro substituiert sein kann oder einen Pyridylrest und
$R_3$ Wasserstoff oder einen Alkylrest;
$R_4$ Wasserstoff oder einen Alkylrest bedeuten oder beide Reste $R_3$ und $R_4$ zusammen mit dem Stickstoffatom einen gesättigten 5- oder 6-Ring, der als weiteres Heteroatom ein O- oder N-Atom enthalten und gegebenenfalls durch Alkyl, bevorzugt Methyl, substituiert sein kann oder einen Imidazolring bilden und wobei

die Aminoalkylgruppe in 4- oder 5-Stellung steht sowie gegebenenfalls ihrer pharmakologisch verträglichen Säureadditionssalze.

2. Mittel nach Anspruch 1, dadurch gekennzeichnet, daß das Antidepressivum [A] aus der Gruppe Desipramin, Clomipramin, Amitryptylin, Nortriptylin, Doxepin, Maprotilin und Imipramin ausgewählt ist.

3. Mittel nach Anspruch 1 oder 2, dadurch gekennzeichnet, daß es eine Verbindung der allgemeinen Formel I enthält, worin $R_1$ Wasserstoff, $R_2$ einen gegebenenfalls durch Fluor, Chlor, Methyl oder Methoxy in o- oder p-Stellung substituierten Phenylrest und $R_3$ und $R_4$,die gleich oder verschieden sein können, Wasserstoff, Methyl oder Ethyl oder $R_3$ und $R_4$ zusammen Morpholin, N-Methylpiperazin oder Pyrrolidin bedeuten können.

4. Mittel nach einem der Ansprüche 1 bis 3, dadurch gekennzeichnet, daß $R_1$ Wasserstoff, $R_3$ Wasserstoff, Methyl oder Ethyl und $R_4$ Wasserstoff, Methyl oder Ethyl bedeuten können.

5. Mittel nach einem der Ansprüche 1 bis 4, dadurch gekennzeichnet, daß $R_3$ und $R_4$ beide Wasserstoff bedeuten.

6. Mittel nach Anspruch 1,dadurch gekennzeichnet, daß es Imipramin und 1-Benzyl-4-aminomethyl-pyrrolidin-2-on bzw. deren Säureadditionssalze enthält.

7. Galenische Zubereitung bestehend aus üblichen Hilfs-und Trägerstoffen, dadurch gekennzeichnet, daß sie eine Kombination gemäß einem der Ansprüche 1 bis 6 enthält.

8. Verwendung eines Mittels gemäß einem der Ansprüche 1 bis 7 als Antidepressivum.

9. Verwendung eines Mittels gemäß einem der Ansprüche 1 bis 7 zur Herstellung eines Arzneimittels mit antidepressiver Wirkung.

10. Verfahren zur Herstellung einer pharmazeutischen Zubereitung mit antidepressiver Wirkung, dadurch gekennzeichnet, daß neben üblichen pharmazeutischen Hilfs- und Trägerstoffen ein Mittel gemäß einem der Ansprüche 1 bis 6 verwendet wird.

## Claims

1. Agent containing an antidepressant [A] of the structural type of a 10,11-dihydro-dibenzo[b,f]-azepine, a dibenzo[a,d][1,4]cycloheptadiene and/or a [10,11]-dihydro-dibenzo-[b,f]oxepine and optionally the harmless acid addition salts thereof and a compound of general formula I [B]

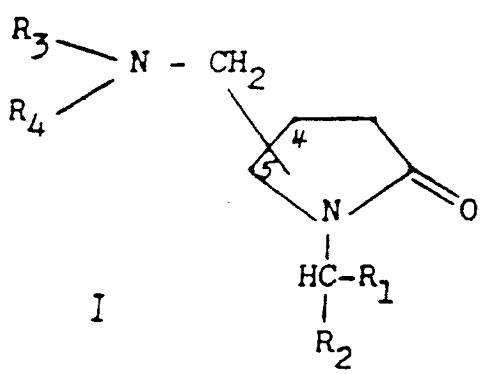

I

wherein

$R_1$ represents hydrogen or an alkyl group,
$R_2$ represents a phenyl group which may be substituted by alkoxy, fluorine, chlorine, bromine, trifluoromethyl, alkyl, hydroxy or nitro, or it may represent a pyridyl group, and
$R_3$ represents hydrogen or an alkyl group;
$R_4$ represents hydrogen or an alkyl group
or the two groups $R_3$ and $R_4$ together with the nitrogen atom may represent a saturated 5- or 6-membered ring which may contain an O or N atom as a further heteroatom and may optionally be substituted by alkyl, preferably methyl, or form an imidazole ring, whilst the amino alkyl group is in the 4- or 5-position, and optionally the pharmacologically acceptable acid addition salts thereof.

2. Agent as claimed in claim 1, characterised in that the antidepressant [A] is selected from the group comprising desipramine, clomipramine, amitryptyline, nortriptyline, doxepine, maprotiline and imipramine.

3. Agent as claimed in claim 1 or 2, characterised in that it contains a compound of general formula I wherein $R_1$ represents hydrogen, $R_2$ represents a phenyl group optionally substituted in the o- or p-position by fluorine, chlorine, methyl or methoxy and $R_3$ and $R_4$, which may be identical or different, represent hydrogen, methyl or ethyl or $R_3$ and $R_4$ together represent morpholine, N-methylpiperazine

or pyrrolidine.

4. Agent as claimed in one of claims 1 to 3, characterised in that $R_1$ may represent hydrogen, $R_3$ may represent hydrogen, methyl or ethyl and $R_4$ may represent hydrogen, methyl or ethyl.

5. Agent as claimed in one of claims 1 to 4, characterised in that $R_3$ and $R_4$ both represent hydrogen.

6. Agent as claimed in claim 1, characterised in that it contains imipramine and 1-benzyl-4-aminomethyl-pyrrolidin-2-one or the acid addition salts thereof.

7. Galenic preparation consisting of conventional excipients and carriers, characterised in that it contains a combination according to one of claims 1 to 6.

8. Use of an agent as claimed in one of claims 1 to 7 as an antidepressant.

9. Use of an agent as claimed in one of claims 1 to 7 for the preparation of a pharmaceutical composition with an antidepressant activity.

10. Process for preparing a pharmaceutical preparation with an antidepressant activity, characterised in that, in addition to conventional pharmaceutical excipients and carriers, an agent as claimed in one of claims 1 to 6 is used.

**Revendications**

1. Agent contenant un antidépresseur [A] de structure du type d'une 10,11-dihydro-dibenzo[b,f]azépine, d'un dibenzo[a,d][1,4] cycloheptadiène et/ou d'une [10,11] - dihydro-dibenzo-[b,f]oxépine ainsi éventuellement que leurs sels d'addition d'acide et un composé [B] de formule générale I

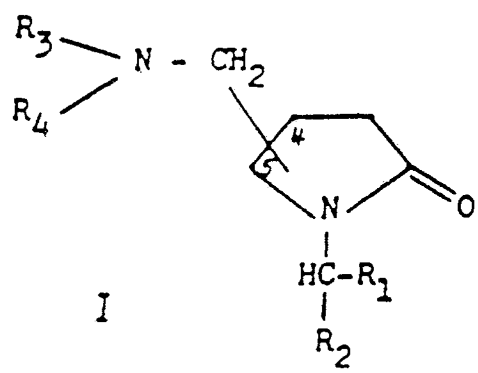

I

dans laquelle
$R_1$ représente un atome d'hydrogène ou un reste alkyle,
$R_2$ représente un reste phényle, qui peut être substitué par alkoxy, fluor, chlore, brome, trifluorométhyle, alkyle, hydroxy ou nitro, ou un reste pyridyle et
$R_3$ représente un atome d'hydrogène ou un reste alkyle;
$R_4$ représente un atome d'hydrogène ou un reste alkyle, ou bien les deux restes $R_3$ et $R_4$ forment avec l'atome d'azote un cycle saturé à 5 ou 6 chaînons qui peut présenter comme hétéroatome supplémentaire un atome d'oxygène ou d'azote et qui peut être éventuellement substitué par alkyle, de préférence méthyle, ou bien ils forment un cycle imidazole, et le groupe aminoalkyle étant en position 4 ou 5, ainsi qu'éventuellement leurs sels d'addition d'acide compatibles du point de vue pharmacologique.

2. Agent selon la revendication 1, caractérisé en ce que l'antidépresseur [A] est choisi dans le groupe formé par la désipramine, la clomipramine, l'amitryptyline, la nortriptyline, la doxépine, la maprotiline et l'imipramine.

3. Agent selon la revendication 1 ou 2, caractérisé en ce qu'il contient un composé de formule générale I

dans laquelle $R_1$ représente un atome d'hydrogène, $R_2$ représente un reste phényle éventuellement substitué par fluor, chlore, méthyle ou méthoxy en position o ou p et $R_3$ et $R_4$, qui peuvent être identiques ou différents, peuvent représenter un atome d'hydrogène, un groupe méthyle ou éthyle ou bien $R_3$ et $R_4$ peuvent représenter ensemble la morpholine, la N-méthylpipérazine ou la pyrrolidine.

**4.** Agent selon l'une des revendications 1 à 3, caractérisé en ce que $R_1$ peut représenter un atome d'hydrogène, $R_3$ peut représenter un atome d'hydrogène, ou un groupe méthyle ou éthyle et $R_4$ peut représenter un atome d'hydrogène, ou un groupe méthyle ou éthyle.

**5.** Agent selon l'une des revendications 1 à 4, caractérisé en ce que $R_3$ et $R_4$ représentent chacun un atome d'hydrogène.

**6.** Agent selon la revendication 1, caractérisé en ce qu'il contient de l'imipramine ou de la 1-benzyl-4-aminométhyl-2-pyrrolidinone ou des sels d'addition d'acide de celles-ci.

**7.** Préparation galénique consistant en adjuvants et véhicules habituels, caractérisée en ce qu'elle contient une combinaison selon l'une des revendications 1 à 6.

**8.** Utilisation d'un agent selon l'une des revendications 1 à 7 comme antidépresseur.

**9.** Utilisation d'un agent selon l'une des revendications 1 à 7 pour la préparation d'un médicament à action antidépressive.

**10.** Procédé de préparation d'une préparation pharmaceutique à action antidépressive, caractérisé en ce que l'on utilise, outre des adjuvants et véhicules pharmaceutiques habituels, un agent selon l'une des revendications 1 à 6.